(19) **Europäisches Patentamt**
**European Patent Office**
**Office européen des brevets**

(11) **EP 1 632 501 B1**

(12) **FASCICULE DE BREVET EUROPEEN**

(45) Date de publication et mention
de la délivrance du brevet:
**25.12.2013 Bulletin 2013/52**

(51) Int Cl.:
*A61K 38/00* (2006.01)    *C07K 14/755* (2006.01)
*C07K 1/18* (2006.01)

(21) Numéro de dépôt: **05291553.5**

(22) Date de dépôt: **20.07.2005**

(54) **Procédé de préparation d'un concentré de facteur von Willebrand (FvW) par voie chromatographique et concentré de FvW susceptible d'être ainsi obtenu**

Prozess zur Herstellung eines Konzentrats des von Willebrand Faktors durch Chromatographie und Konzentrat des durch diesen Prozess erhaltenen von Willebrand Faktors

Process for preparing a concentrate of von Willebrand factor with chromatography and concentrate of von Willebrand factor obtained according to said process

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **16.08.2004 FR 0408897**

(43) Date de publication de la demande:
**08.03.2006 Bulletin 2006/10**

(60) Demande divisionnaire:
**10186193.8 / 2 292 656**

(73) Titulaire: **Laboratoire Français du Fractionnement et des Biotechnologies**
**91940 Les Ulis (FR)**

(72) Inventeurs:
• **Martel, Serge**
  **59110 La Madeleine (FR)**
• **Chtourou, Sami**
  **78990 Elancourt (FR)**
• **Poulle, Michel**
  **59136 Wavrin (FR)**

(74) Mandataire: **Hirsch & Associés**
**58, avenue Marceau**
**75008 Paris (FR)**

(56) Documents cités:
**EP-A- 0 359 593**    **EP-A- 0 416 983**
**EP-A- 0 503 991**    **EP-A- 0 934 748**
**EP-A1- 0 469 985**    **EP-A2- 0 383 234**
**US-A- 5 854 403**

• **BURNOUF T: "Chromatography in plasma fractionation: benefits and future trends" JOURNAL OF CHROMATOGRAPHY B : BIOMEDICAL APPLICATIONS, ELSEVIER SCIENCE PUBLISHERS, NL, vol. 664, no. 1, 3 février 1995 (1995-02-03), pages 3-15, XP004043701 ISSN: 0378-4347**

Remarques:
Le dossier contient des informations techniques présentées postérieurement au dépôt de la demande et ne figurant pas dans le présent fascicule.

**Description**

La présente invention concerne un procédé de préparation d'un concentré de facteur von Willebrand (FvW) par voie chromatographique.

Le facteur von Willebrand est une protéine sanguine multimérique dont les poids moléculaires se situent entre environ 200 kDa et environ 20 000 kDa, voire davantage. Cette protéine, synthétisée par les plaquettes et les cellules endothéliales, joue un rôle primordial dans la lutte contre l'hémorragie sanguine dans la mesure où le FvW agit comme un tampon gélifiable qui s'étale sur une brèche vasculaire fournissant l'adhésion des plaquettes pour réaliser la première phase de l'hémostase, à savoir la formation du "clou plaquettaire" (thrombus). Autour de ce thrombus vont s'organiser les phénomènes de la coagulation destinés à consolider l'arrêt du saignement par formation d'un caillot de fibrine insoluble. Le FvW assure également dans la circulation sanguine la stabilisation et le transport du Facteur VIII, auquel il est associé sous forme de complexes de tailles variables, lequel se trouve ainsi protégé contre une dégradation rapide par protéolyse en raison de la sensibilité du Facteur VIII isolé aux protéases.

Une déficience congénitale en FvW ou des mutations génétiques modifiant les propriétés du FvW entraîne la maladie de Willebrand qui se manifeste donc par des troubles de l'hémostase primaire et de la coagulation.

Il est par conséquent essentiel, dans le traitement de cette maladie, de disposer de dérivés de plasma humain enrichis en FvW de très grande pureté compatibles avec des injections multiples et répétées. En effet, des échantillons insuffisamment purifiés de FvW, provenant du fractionnement du plasma humain, contiennent divers contaminants (protéines résiduelles) pouvant induire des réponses immunologiques indésirables. De plus, l'administration de facteur von Willebrand associé à du Facteur VIII peut entraîner un risque de thrombose ou d'hypercoagulabilité pour le patient traité (Makris et al, Thromb.Haemost, 88, 2002, pp.377-378, Manucci P.M., Thromb.Haemost, 88, 2002, pp.378-379).

Divers procédés de préparation de concentrés de FvW associent typiquement des étapes de précipitation d'une fraction de plasma, destinée à l'élimination de la majeure partie des protéines indésirables (fibrinogène, fibronectine etc.), et/ou de chromatographies (échange d'ions, d'affinité, d'immunoaffinité, d'exclusion stérique etc.) qui visent à l'obtention de concentrés de très grande pureté, présentant une activité spécifique élevée, et qui permettent de conserver l'intégrité des formes multimériques, notamment celles de haut poids moléculaire dont l'importance biologique est fondamentale dans les processus de cicatrisation.

A titre d'exemple, on peut citer le brevet EP 0 359 593 qui divulgue la séparation de protéines d'une fraction de plasma cryoprécipité mettant en oeuvre plusieurs étapes de chromatographie d'échange d'anions conduisant à la purification du FvW.

Le brevet EP 0 503 991 divulgue un procédé de préparation à l'échelle industrielle d'un concentré de FvW comprenant une étape de prépurification d'une fraction cryoprécipitée de plasma et trois étapes successives de chromatographie, la troisième étant une chromatographie d'affinité sur colonne de gélatine immobilisée sur agarose. Le concentré de FvW ainsi obtenu présente une activité spécifique supérieure à 100 UI RCo/mg exprimée en unités d'activité de cofacteur de ristocétine par mg de protéines et un taux de multimères de hauts poids moléculaire comparable à celui du plasma de départ.

La demande de brevet EP 0 934 748 décrit un procédé de préparation de FvW comprenant la combinaison de chromatographies d'échange d'anions et d'échange de cations. Les fractions de FvW obtenues présentent une activité spécifique supérieure à 100 UI FvW:Ag/mg exprimée en unités d'antigène de FvW par mg de protéine, mais contiennent des proportions encore notables de Facteur VIII.

Le brevet US 6 579 723 décrit un procédé de préparation d'un FvW hautement purifié par chromatographie d'immunoaffinité dont les immunoadsorbants représentent des anticorps anti-FvW. Il peut également être prévu une étape supplémentaire de purification par chromatographie d'affinité sur héparine. Toutefois, l'inconvénient d'une purification par immunoaffinité est la présence éventuelle d'anticorps résiduels pouvant entraîner des réactions immunologiques.

Le brevet EP 0 383 234 enseigne la préparation d'un concentré de FvW pasteurisé par une chromatographie d'échange d'anions, mise en oeuvre avec des solutions acides (pH de 5,5 à 6,5) contenant des hydrates de carbone, pour la fixation du Facteur VIII sur l'échangeur d'anions. La récupération conjointe du FvW, de la fibronectine et du fibrinogène non retenus, par lavage du support, nécessite des étapes supplémentaires de précipitation pour l'isolement d'un concentré de FvW purifié.

Les procédés décrits ci-dessus présentent l'inconvénient de nécessiter plusieurs étapes successives, notamment chromatographiques, ce qui pose des problèmes de rendement et de lourdeur de mise en oeuvre à l'échelle industrielle. Elles peuvent utiliser, selon les cas, des supports chromatographiques à ligands d'origine animale, tels qu'à base de gélatine, d'héparine ou de collagène susceptibles d'être le véhicule de prion pathogène, responsable d'encéphalopathie spongiforme ou d'autres virus propres à l'espèce animale considérée. Ces difficultés sont encore amplifiées par la nécessité d'inclure dans le procédé une inactivation virale et, le cas échéant, des étapes supplémentaires d'élimination d'agents virucides. Par ailleurs, les concentrés de FvW obtenus par ces procédés ne sont pas exempts de Facteur VIII, ce qui présente l'inconvénient de risques de thrombose chez des patients. En outre, le nettoyage ou lavage des supports d'affinité ainsi que leur sanitisation reste difficile en raison de la fragilité du ligand qui empêche l'utilisation de solutions de nettoyage à caractère désinfectant fort (eau de Javel, soude, potasse etc.). Enfin, les supports d'affinité ont des durées de vie assez courtes et leur remplacement fréquent représente une charge financière importante qui grève le prix de revient du produit traité.

Compte tenu des besoins sans cesse croissants en fractions de FvW très pures, la Demanderesse s'est attachée à mettre au point un procédé de préparation de FvW, dont la mise en oeuvre est très simple, à haut rendement, ne nécessitant pas l'utilisation de support chromatographique à ligands d'origine animale et conduisant à un concentré de FvW de grande pureté, standardisé, à très haute activité spécifique et exempt de Facteur VIII.

A cet effet, l'invention concerne un procédé de préparation d'un concentré de facteur von Willebrand de très haute pureté à partir d'une fraction biologique contenant du facteur von Willebrand, caractérisé en ce qu'il comprend une séparation par chromatographie d'échange d'anions utilisant un support de polymère vinylique, de type base faible, ladite séparation comportant les étapes de

a) - chargement du support chromatographique en fraction contenant du facteur von Willebrand, préalablement équilibré par un tampon approprié, à un débit prédéterminé, ce qui permet la rétention du facteur von Willebrand ;
b) - lavage du support par un tampon acide avec un débit supérieur à celui de l'étape a) jusqu'à l'élimination de protéines et de contaminants non retenus ;
c) - rinçage et équilibrage du support chromatographique avec le tampon et le débit de l'étape a) ; et
d) - élution du facteur von Willebrand par augmentation de la force ionique du tampon de l'étape c).

Ainsi, la Demanderesse a trouvé qu'il était possible grâce à un procédé très simple d'aboutir à un concentré de FvW de qualité accrue, de haute activité spécifique et essentiellement exempt de Facteur VIII. Elle a pu plus précisément montrer que le choix judicieux du support chromatographique échangeur d'anions de type polymère vinylique et des conditions physico-chimiques particulières de lavage de ce support (pH acide, débit accru) permettait de séparer, en une seule étape, le FvW retenu par le support, du reste des protéines, notamment le Facteur VIII, et autres composés contaminants présents dans la fraction biologique contenant le FvW.

Avantageusement, la séparation chromatographique est effectuée sur un support synthétique, résine ou gel, dont la matrice est de type polymère vinylique, représentant plus préférablement un copolymère d'oligoéthylèneglycol, de glycidylméthacrylate et de pentaérythritoldiméthacrylate, sur lequel sont greffés des groupements échangeurs d'anions de type base faible, tels que le DEAE. De telles matrices vinyliques sont connues sous l'appellation Fractogel®-TSK et, dans le cadre de l'invention, les supports échangeurs d'anions représentent notamment le DEAE-Fractogel®-TSK 650, disponible sous deux granulométrie M (moyenne performance) et S (haute performance), et dont les utilisations ont fait l'objet de nombreuses études. Le support chromatographique se présente habituellement sous forme de colonnes chromatographiques de dimensions adaptées selon les applications désirées (colonne analytique ou préparative).

La Demanderesse a donc mis à profit les caractéristiques de rigidité et de grande taille des pores de ce support pour la mise en oeuvre du procédé qui permettaient d'empêcher l'accumulation de certaines protéines ayant tendance à précipiter sous des conditions acides pendant la chromatographie, telles que la fibronectine et le fibrinogène, évitant ainsi le colmatage du support, sans avoir à recourir à l'ajout de quantités importantes de sucre, comme décrit dans EP 0 383 234. A cet effet, la non accumulation de ces protéines est rendue possible notamment par une augmentation du débit pendant la phase du lavage, grâce également à un temps de relaxation important de celles-ci pour se mettre aux conditions de pH du tampon acide, compte tenu de leur masse moléculaire (> 300 kDa), et du fait de leur faible teneur dans les échantillons de départ, ce qui limite la probabilité de rencontre pouvant aboutir à la formation de polymères ou d'agrégats in situ.

Dans le cadre de l'invention, la fraction biologique contenant du facteur von Willebrand représente soit du plasma humain soit une fraction de plasma humain cryoprécipité ou du surnageant de plasma cryoprécipité contenant encore du FvW ou obtenue par des méthodes classiques de fractionnement (Cohn et al, J. Am. Chem. Soc., 68, 459, 1946 et Kistler et al, Vox Sang., 7, 1962, 414-424), ayant éventuellement subi un traitement de prépurification tel que par adsorption sur hydroxyde d'aluminium, dans laquelle le FvW est complexé au Facteur VIII, ou bien encore une fraction enrichie en complexe Facteur VIII/FvW recombinant isolé à partir de surnageant de cultures cellulaires selon des techniques connues ou enfin de complexe Facteur VIII/FvW obtenu dans le lait de mammifères transgéniques comme décrit dans le brevet US 6 518 482. De façon particulièrement préférée, la fraction de départ représente une fraction de FvW obtenue par une étape préalable de purification d'une fraction du plasma cryoprécipité par voie chromatographique utilisant un échangeur d'anions de type DEAE-Fractogel®-TSK 650, tel que décrit dans les brevets EP 0 359 593 et EP 0 503 991. Cette étape préalable offre l'avantage de permettre la récupération de la majeure partie des protéines d'intérêt telles que le Facteur VIII, le fibrinogène et la fibronectine.

Le tampon d'équilibrage (étape a) ou c)) est composé de chlorure de sodium, dont la concentration est de préférence de 0,11 M, et peut en outre comprendre du citrate trisodique, du chlorure de calcium, de la glycine et de la lysine, à un pH de 6,9-7,1, dont les concentrations en chacun des composants représentent respectivement, de façon préférée, 0,01 M, 0,001 M, 0,12 M et 0,016 M. On peut également utiliser tout autre tampon, à base de chlorure de sodium, comprenant d'autres composés biologiquement compatibles à condition qu'ils ne dénaturent pas de façon irréversible le FvW.

Quand la fraction contenant du FvW a été appliquée sur le support chromatographique, on procède à son lavage par percolation du tampon acide (étape b)). Le tampon acide est avantageusement composé d'un sel alcalin ou alcalino-terreux de l'acide acétique, citrique ou phosphorique, en une concentration comprise entre 10 et 30 mM, de pH compris entre 3,9 et 5,2, et représente de préférence un tampon acétate de sodium 20 mM de pH 4,35 environ. Cette étape de lavage permet le passage dans le filtrat de protéines présentes dans la fraction biologique de départ, telles que la fibronectine, le fibrinogène, le Facteur VIII etc., et des contaminants non retenus ou faiblement retenus par le support. Elle est effectuée par augmentation du débit mis en oeuvre à l'étape a). De cette façon, on évite l'accumulation des protéines et des contaminants ayant tendance à précipiter, sans le besoin d'ajouter des sucres qu'il serait ensuite nécessaire d'éliminer. Le débit de la présente étape de lavage est judicieusement choisi pour obtenir l'effet recherché en veillant à ce que celui-ci n'altère pas les propriétés physico-chimiques du support chromatographique par des valeurs excessives. De préférence, le débit de lavage correspond à une valeur supérieure à celle de l'étape de d'équilibrage a) d'un facteur d'environ 1,5 à 2. La durée du lavage est déterminée par mesure de la densité optique (DO) du filtrat à la longueur d'onde de 280 nm. En effet, une valeur de DO correspondant à celle de la ligne de base est une bonne indication de l'élimination effective des composés cités ci-dessus à partir du support et leur sortie de la colonne chromatographique.

Après retour à la ligne de base, on diminue le débit à la valeur de celui de l'étape a) et l'élution du FvW (étape d)) est effectuée par l'utilisation du tampon de l'étape a) dont la force ionique est augmentée. Cette augmentation de la force ionique est effectuée avantageusement par ajout de chlorure de sodium dont la concentration finale est portée à 0,15-0,17 M. L'utilisation selon l'invention du support chromatographique de matrice polymère vinylique selon l'invention, de nature légèrement hydrophobe, permet la séparation du FvW des impuretés et/ou des protéines accompagnantes, telles que la fibronectine. De façon surprenante, le FvW obtenu par le procédé est également dépourvu de Facteur VIII.

Le procédé selon la présente invention peut être adapté à des volumes de plasma d'environ 4000 litres.

Le procédé peut comporter au moins une étape de traitement d'inactivation virale de la fraction contenant du FvW à purifier. Ainsi, celle-ci peut être soumise à un traitement classique d'inactivation virale par solvant-détergent, en présence d'agents d'inactivation, tels que de Tween®-TNBP, avant l'étape chromatographique, cette dernière permettant d'éliminer totalement les produits résiduels de cette étape de décontamination.

En particulier, une fois la fraction de FvW récoltée, elle peut être soumise à divers traitements destinés à la rendre de qualité thérapeutique. Ainsi, après l'étape d), le procédé peut comprendre une ou plusieurs étapes supplémentaires de filtration stérilisante conventionnelle puis de filtration d'élimination virale, telle que nanofiltration sur un filtre de 35 nm. Ensuite, la fraction de FvW peut être diafiltrée afin d'incorporer des excipients adéquats destinés à autoriser un chauffage à sec du FvW sans risque de dénaturation, concentrée par ultrafiltration, conditionnée en flacons et lyophilisée, après un ajout préalable d'un stabilisant supplémentaire pharmaceutiquement acceptable, tel que l'albumine. Enfin, les lyophilisats subissent une ultime étape d'inactivation virale par chauffage à sec du lyophilisat selon des conditions classiques, à 80°C pendant 72 heures, pour l'inactivation des virus non enveloppées qui n'auraient pas été inactivés et/ou éliminés par au moins l'une des deux étapes d'inactivation et/ou d'élimination virales précédentes. Les lyophilisats chauffés à sec sont ensuite reconstitués dans un milieu aqueux compatible à un usage clinique, de préférence dans 10 ml d'eau purifiée pour injection (PPI) et peuvent être directement injectés par voie intraveineuse.

Ainsi, la mise en oeuvre du procédé, après l'étape d'ultrafiltration, conduit à un concentré de FvW hautement purifié, de qualité thérapeutique, présentant une activité spécifique (A.S.) d'au moins 90 UI RCo/mg de protéine. En outre, le rapport R, représentant Facteur VIII:C/FvW: RCo, est inférieur à 0,06%. Ce résultat révèle que le concentré de FvW est dépourvu de Facteur VIII ou n'en contient que des doses infimes.

Le degré total de purification du présent procédé est supérieur à 10 000, lorsqu'une fraction biologique de plasma est utilisée.

Le produit final de FvW obtenu, c'est à dire le lyophilisat de FvW chauffé reconstitué dans de l'eau PPI, présente un taux de multimères, mesuré en référence à la méthode 0275 de la Pharmacopée Européenne, comparable à celui du plasma qui est de 70%. Les taux résiduels en TNBP et Tween® sont également conformes aux valeurs fixées par la Pharmacopée Européenne.

La stabilité du concentré de FvW (après ultrafiltration) a été étudiée pendant 24 heures à température ambiante : on ne détecte aucune trace de protéolyse. En outre, le produit final lyophilisé de FvW reste stable sur une période de stockage d'environ deux ans à 30°C et six mois à 40°C. Il présente une capacité de liaison au Facteur VIII similaire à celui du FvW présent dans le plasma natif.

Les exemples qui suivent illustrent des modes de réalisation de la présente invention sans toutefois en limiter la portée.

Exemple 1

1) Obtention d'une fraction contenant du FvW

On utilise un cryoprécipité de plasma humain, remis en suspension dans une solution aqueuse d'héparine sodique (à 2 U/ml), à pH de 7-7,1.

Cette suspension de cryoprécipité est soumise à une prépurification sur hydroxyde d'aluminium pour l'élimination des principaux contaminants, telle que décrite dans le brevet EP 359 593. Le surnageant prépurifié est ensuite récupéré et est soumis à un traitement d'inactivation virale classique par solvant-détergent, en présence de Tween®-TNBP.

La solution de cryoprécipité prépurifié est ensuite injectée sur une colonne chromatographique de Fractogel® TSK-DEAE 650 (M) de 25 cm de longueur et de 1 cm de diamètre, préalablement équilibrée en tampon constitué de citrate trisodique 0,01 M, de chlorure de calcium 0,001 M, de chlorure de sodium 0,11 M, de glycine 0,12 M et de lysine 0,016 M, ajusté à pH 7,01, dont la vitesse linéaire de la phase mobile est fixée à 100 cm/heure. Le FvW, le Facteur VIII et la fibronectine sont retenus par le support chromatographique. Les protéines faiblement ou pas retenues par le support, principalement le fibrinogène et des IgG, sont éliminées dans le filtrat, ainsi que les Tween® et TNBP, par plusieurs lavages successifs avec le même tampon.

Lorsque la DO, mesurée à 280 nm, est retombée à la valeur de celle de la ligne de base, on augmente la concentration en chlorure de sodium du tampon à 0,15 M. Dans ces conditions, le FvW est élué. L'éluat ainsi obtenu est très enrichi en FvW et en fibronectine et contient encore du Tween® et du TNBP, ainsi que du Facteur VIII résiduel.

2) Séparation chromatographique

La fraction enrichie en FvW éluée précédemment, constituant un lot de fraction de départ contenant le FvW selon l'invention, est chargée sur une colonne chromatographique de DEAE-Fractogel®-TSK 650 (M) de 25 cm de longueur et de 1 cm de diamètre, préalablement équilibrée en tampon identique à celui de 1), d'osmolarité de 387 mosmolkg$^{-1}$, dont la vitesse linéaire est fixée à 100 cm/heure. On injecte 140 ml de cette fraction contenant 12,9 UI FvW/ml et 6,6 UI Facteur VIII/ml, soit un rapport R de 51,1% (R= FVIII:C/FvW:RCo).

La colonne est ensuite lavée par un tampon acide d'acétate de sodium 20 mM, ajusté à pH 4,35 et 80 mosmolkg$^{-1}$, avec une vitesse linéaire de 150 cm/heure. Dans ces conditions, on assure une très bonne élimination non seulement des résidus d'agents d'inactivation virale, mais également de la fibronectine et surtout du Facteur VIII qui était encore complexé au FvW, sans observer de précipitation de ces protéines dans la colonne. Lorsque la DO est retombée à la valeur de celle de la ligne de base, on ramène la vitesse linéaire à 100 cm/heure puis on rince la colonne et on l'équilibre avec le même tampon que précédemment, contenant du NaCl à 0,11 M.

L'élution de la fraction contenant le FvW s'effectue par augmentation de la concentration en NaCl du tampon d'équilibrage à 0,17 M, ajusté à un pH de 6,95 et 492 mosmolkg$^{-1}$.

La fraction éluée de FvW subit ensuite les traitements classiques de filtration stérilisante sur filtres de 0,22 $\mu$m, de nanofiltration sur des filtres de 35 nm, de diafiltration contre une solution contenant de l'arginine, au moins un acide aminé hydrophobe et du citrate trisodique, telle que décrite par la Demanderesse dans la demande de brevet FR 03 08403, et d'ultrafiltration selon des techniques connues de façon à ce que le concentré de FvW présente une activité spécifique (A.S.) d'au moins 90 UI RCo/mg de protéine.

Les concentrés de FvW ainsi obtenus sont additionnés d'albumine à 10 g/l puis lyophilisés à -40°C pendant 48 heures. La lyophilisation est suivie d'un traitement thermique d'inactivation virale par chauffage à sec du lyophilisat à 80°C pendant 72 heures.

Afin de comparer les performances du procédé selon l'invention par la qualité du concentré de FvW obtenu en terme d'activité spécifique, de teneur résiduelle en divers contaminants protéiniques, notamment de Facteur VIII, des comparaisons ont été effectuées avec le concentré de FvW obtenu par la mise en oeuvre du procédé décrit dans le brevet EP 0 503 991, noté procédé A.

La mise en oeuvre du procédé selon ce brevet comprend les étapes suivantes consistant à :

- faire passer un lot de fraction de FvW issue de la première séparation chromatographique décrite en 1) sur une deuxième colonne chromatographique de 25 cm de longueur et de 1 cm de diamètre contenant du DEAE-Fractogel®-TSK 650 (M) dans les conditions de mise en oeuvre telles que décrites en 1) (tampon, vitesse linéaire identiques) ;
- éliminer le filtrat et rincer la colonne avec le tampon d'équilibrage,
- éluer le FvW par augmentation de la concentration en NaCl du tampon d'équilibrage à 0,17 M ;
- soumettre l'éluat de FvW à une étape de purification supplémentaire sur une colonne dont le support chromatographique est du type gélatine immobilisée sur agarose équilibré en tampon d'équilibrage destinée à éliminer la fibronectine résiduelle.

L'éluat de cette ultime étape chromatographique subit ensuite des traitements identiques de filtration stérilisante, filtration d'élimination virale, de diafiltration, d'ultrafiltration, d'ajout d'albumine, de lyophilisation et de chauffage à sec.

Le Tableau 1 présente les résultats des rendements de préparation de fractions de FvW, exprimés en UI RCo/mg de protéine, à divers stades du procédé selon l'invention et selon A, à savoir après toutes les chromatographies, ultrafiltration, et après lyophilisation, chauffage à sec (80°C, 72 heures) et reconstitution dans 10 ml d'eau purifiée pour injection (PPI). On fait subir les mêmes traitements d'ultrafiltration et de chauffage à sec du lyophilisat de la fraction de FvW de départ suivi d'une remise en solution dans 10 ml d'eau PPI. Les rendements sont exprimés sous la forme de rapport :

$$R1 = \frac{\text{A.S. de la fraction de FvW de départ}}{\text{A.S. de la fraction de FvW après les chromatographies}}$$

$$R2 = \underline{\text{A.S. de la fraction de FvW de départ ultrafiltrée}}$$

$$R3 = \frac{\text{A.S. du lyophilisat de FvW de départ chauffé}}{\text{A.S. du lyophilisat de FvW chauffé}}$$

Les A.S. (UI RCo/mg), mesurées sur le concentré de FvW et les teneurs en fibronectine résiduelle (TFR) correspondantes sont également indiquées.

Les valeurs données représentent les valeurs moyennes de six essais.

Tableau 1

| n = 6 | R1 (%) | R2 (%) | R3 (%) | As* | TFR μg/UIFvW:R Co |
|---|---|---|---|---|---|
| fraction de FvW selon l'invention | 87±5 | 97,9±5,2 | 84,1±6,1 | 98,7±5,6 | < 0,04 |
| fraction de FvW selon A | - | 89,4±5,7 | 84,1±11,6 | 98,3±14,5 | < 0,04 |
| * : A.S. obtenue sur le concentré de FvW | | | | | |

Les deux procédés sont comparables et donnent des concentrés de FvW d'un rendement satisfaisant et d'une A.S. élevées dans les deux cas.

Le Tableau 2 présente les résultats des différents essais destinés à évaluer le taux de contaminants résiduels protéiniques et chimiques présents dans un lyophilisat de FvW chauffé à sec et remis en solution dans 10 ml d'eau PPI, obtenu selon l'invention (Produit I) et selon A (Produit II). Les valeurs données représentent les valeurs moyennes de six essais.

Tableau 2

| | Produit I | Produit II |
|---|---|---|
| Activité FVIII:C (UI/ml) | 0,035 ± 0,025 | 1,8 à 9,2 |
| R | 0,035 ± 0,025 | 1,84 à 8,85 |
| Teneur protéinique (g/l) | 9-10 | 11,25 ± 0,25 |
| Teneur en Tween® (mg/l) | < 20 | < 20 |
| Teneur en TNBP (mg/l) | < 0,1 | < 0,1 |

Entre le concentré obtenu selon le procédé A et celui obtenu selon l'invention, apparaît une différence considérable quant au rapport R (FVIII:C/FvW:RCo). En effet, le concentré obtenu selon l'invention est quasiment exempt de FVIII:C dosable.

Le Tableau 3 présente les résultats des rendements, des activités spécifiques et des teneurs en fibronectine résiduelle, obtenus à partir d'un même lot de fraction de départ de FvW, divisé en deux fractions égales de FvW, dont l'une est soumise au procédé selon l'invention et l'autre fraction est soumise au procédé selon A. Les rendements sont mesurés à différents stades des procédés, selon les conditions décrites pour les résultats du Tableau 1. Les valeurs données représentent les valeurs moyennes de six essais.

Tableau 3

| n = 6 | R1 (%) | R2 (%) | R3 (%) | As* | TFR $\mu$g/UIFvW:R Co |
|---|---|---|---|---|---|
| fraction de FvW selon l'invention | $89 \pm 10$ | $88 \pm 12$ | $86,3 \pm 9,2$ | $98,7 \pm 5,6$ | < 0,04 |
| fraction de FvW selon A | $87 \pm 10$ | $76,3 \pm 7,5$ | $70 \pm 8$ | $98,3 \pm 14,5$ | < 0,04 |
| * : A.S. obtenue sur le concentré de FvW | | | | | |

Les résultats du tableau 3 montrent bien que la qualité du produit final de FvW obtenu par le procédé de l'invention est au moins égale à celle obtenue par le procédé selon A et que les rendements sont comparables.

Le Tableau 4 présente la teneur en diverses protéines présentes dans une fraction de FvW lyophilisée et chauffée à sec et remise en solution dans 10 ml d'eau PPI, obtenue selon le procédé de l'invention (Produit III) et selon le procédé A (Produit IV). Les valeurs données représentent les valeurs moyennes de trois essais.

Tableau 4

| n = 3 | Produit III | Produit IV |
|---|---|---|
| FvW ($\mu$g/ml) | $1022 \pm 57$ | $1015 \pm 72$ |
| FVIII ($\mu$g/ml) | $0,14 \pm 0,08$ | $1,82 \pm 0,53$ |
| Fibrinogène ($\mu$g/ml) | $8,2 \pm 5,2$ | $3,5 \pm 1,2$ |
| Fibronectine ($\mu$g/UI FvW:RCo) | > 0,04 | > 0,04 |
| Immunoglobuline G ($\mu$g/ml) | $1,93 \pm 0,71$ | $4,23 \pm 0,95$ |
| Immunoglobuline A ($\mu$g/ml) | 1,4 | $2,23 \pm 0,46$ |
| Immunoglobuline M ($\mu$g/ml) | < 8,5 | $28,8 \pm 8,9$ |
| Inter-$\alpha$-trypsine inhibiteur ($\mu$g/ml) | $14,83 \pm 5,25$ | $1,93 \pm 0,83$ |
| Plasminogène ($\mu$g/ml) | 0,006 | $0,0106 \pm 0,0022$ |
| Protéine C ($\mu$g/ml) | < 2,5 | < 2,5 |

Les résultats du Tableau 4 montrent que la fraction de FvW selon l'invention présente une teneur nettement inférieure en FVIII et en Immunoglobulines M à celle obtenue par la mise en oeuvre du procédé A. En revanche, elle contient davantage d'inter-$\alpha$-trypsine inhibiteur (ITI), ce qui n'est toutefois pas préjudiciable aux propriétés thérapeutiques du concentré de FvW, contrairement au paramètre teneur de FVIII mesurable.

Exemple 2

On utilise diverses fractions contenant du FvW, obtenues à partir d'un cryoprécipité ayant subi le traitement explicité à l'Exemple 1, 1). Ces fractions de FvW sont ajustées de façon telle qu'elles contiennent un taux variable en rapport R (FVIII:C/FvW:RCo) initial s'échelonnant d'environ 30% à environ 120%.

Ces fractions subissent ensuite une séparation chromatographique selon l'invention et selon le procédé A et les divers traitements conduisant à des lyophilisats chauffés à sec remis en solution dans de l'eau PPI, selon les conditions décrites dans l'Exemple 1, 2).

Les résultats des rendements figurent au Tableau 5.

Tableau 5

| R initial | 32,6% | 51% | 64,6% | 120,8% |
|---|---|---|---|---|
| Rendement FvW selon A (%) | 92,9 | 47,4 | 46,8 | 97,4 |
| Rendement FvW selon l'invention (%) | 93,1 | 53,4 | 95,7 | 104 |
| R selon A (%) | 8,8 | 22,8 | 10,6 | 21,3 |
| R selon l'invention (%) | 0 | 0,05 | 0 | 0 |

La mise en oeuvre du procédé selon l'invention conduit donc à une élimination presque totale du FVIII tout en assurant un rendement de production accru.

**Revendications**

1.  Procédé de préparation d'un concentré de facteur von Willebrand de très haute pureté à partir d'une fraction biologique contenant du facteur von Willebrand, **caractérisé en ce qu'**il comprend une séparation par chromatographie d'échange d'anions utilisant un support de polymère vinylique, de type base faible, ladite séparation comportant les étapes de a) - chargement du support chromatographique en fraction contenant du facteur von Willebrand, préalablement équilibré avec un tampon approprié, à un débit prédéterminé, ce qui permet la rétention du facteur von Willebrand ;

    b) - lavage du support par un tampon acide avec un débit supérieur à celui de l'étape a) jusqu'à l'élimination des protéines et des contaminants non retenus ;
    c) - rinçage et équilibrage du support chromatographique avec le tampon et le débit de l'étape a) ; et
    d) - élution du facteur von Willebrand par augmentation de la force ionique du tampon de l'étape c).

2.  Procédé selon la revendication 1, **caractérisé en ce que** la séparation chromatographique est effectuée sur un support de type polymère vinylique greffé de groupements échangeurs d'anions représentant le DEAE.

3.  Procédé selon la revendication 2, **caractérisé en ce que** le support chromatographique représente un copolymère d'oligoéthlèneglycol, de glycodiylméthacrylate et de pentaérythritoldiméthacrylate sur lequel sont greffés des groupements échangeurs d'anions de type base faible, tels que le DEAE.

4.  Procédé selon l'une quelconque des revendications 1 à 3, **caractérisé en ce que** le tampon d'équilibrage de l'étape a) ou c) est composé de chlorure de sodium

5.  Procédé selon la revendication 4, **caractérisé en ce que** la concentration en chlorure de sodium est de 0,11 M.

6.  Procédé selon la revendication 4 ou 5, **caractérisé en ce que** le tampon d'équilibrage comprend en outre du citrate trisodique, du chlorure de calcium, de la glycine et de la lysine, de pH compris entre 6,9 et 7,1.

7.  Procédé selon la revendication 6, **caractérisé en ce que** les concentrations des composants du tampon d'équilibrage représentent respectivement, 0,01 M, 0,001 M, 0,12 M et 0,016 M.

8.  Procédé selon l'une quelconque des revendications 1 à 7, **caractérisé en ce que** le tampon acide de lavage est composé d'un sel alcalin ou alcalino-terreux de l'acide acétique, citrique ou phosphorique, en une concentration comprise entre 10 et 30 mM et de pH compris entre 3,9 et 5,2.

9.  Procédé selon la revendication 8, **caractérisé en ce que** le tampon acide de lavage représente un tampon acétate de sodium 20 mM de pH 4,35.

10. Procédé selon l'une quelconque des revendications 1 à 9, **caractérisé en ce que** le débit de l'étape de lavage b) correspond à une valeur supérieure à celle de l'étape d'équilibrage a) d'un facteur d'environ 1,5 à 2.

11. Procédé selon l'une quelconque des revendications 1 à 10, **caractérisé en ce que**, pour l'étape d), la force ionique du tampon de l'étape c) est augmentée par ajout de chlorure de sodium dont la concentration finale est portée à 0,15-0,17 M.

12. Procédé selon l'une quelconque des revendications 1 à 11, **caractérisé en ce qu'**il comprend une étape d'inactivation virale de la fraction biologique par solvantdétergent.

13. Procédé selon l'une quelconque des revendications 1 à 12, **caractérisé en ce qu'**il comprend après l'étape d'élution d), une ou plusieurs étapes supplémentaires de

- filtration stérilisante,
- filtration d'élimination virale,
- diafiltration,
- ultrafiltration de concentration,
- ajout d'un stabilisant pharmaceutiquement acceptable,
- lyophilisation,
- chauffage à sec d'inactivation virale et,
- reconstitution du lyophilisat chauffé dans un milieu aqueux compatible à un usage clinique.

**Patentansprüche**

1. Verfahren zur Herstellung eines Konzentrats des von Willebrand Faktors von sehr hoher Reinheit ausgehend von einer biologischen Fraktion, die den von Willebrand Faktor enthält, **dadurch gekennzeichnet, dass** es eine Trennung durch Anionenaustauschchromatographie umfasst, die einen Träger auf Vinylpolymer des schwach basischen Typs verwendet, wobei die Trennung die folgenden Schritte umfasst:

a) - Beladen des Chromatographieträgers mit einer den von Willebrand Faktor enthaltenden Fraktion, die zuvor mit einem geeigneten Puffer äquilibriert wurde, mit einem vorherbestimmten Durchfluss, was das Zurückhalten des von Willebrand Faktors gestattet;
b) - Waschen des Trägers mit einem sauren Puffer mit einem höheren Durchfluss als dem aus Schritt a) bis zur Beseitigung von nicht zurückgehaltenen Proteinen und Verunreinigungen;
c) - Spülen und Äquilibrieren des Chromatographieträgers mit dem Puffer und dem Durchfluss aus Schritt a); sowie
d) - Eluieren des von Willebrand Faktors durch Erhöhen der Ionenstärke des Puffers aus Schritt c).

2. Verfahren nach Anspruch 1, **dadurch gekennzeichnet, dass** die chromatographische Trennung durch einen Träger vom Typ Vinylpolymer bewirkt wird, der mit Anionenaustauschergruppen besetzt ist, die DEAE darstellen.

3. Verfahren nach Anspruch 2, **dadurch gekennzeichnet, dass** der Chromatograhieträger ein Copolymer aus Oligoethylenglykol, Glykodiylmethacrylat und Pentaerythritoldimethacrylat darstellt, auf dem Anionenaustauschergruppen des schwach basischen Typs angeordnet sind, wie etwa DEAE.

4. Verfahren nach einem der Ansprüche 1 bis 3, **dadurch gekennzeichnet, dass** der Äquilibrierungspuffer aus Schritt a) oder c) aus Natriumchlorid besteht.

5. Verfahren nach Anspruch 4, **dadurch gekennzeichnet, dass** die Konzentration an Natriumchlorid 0,11 M beträgt.

6. Verfahren nach Anspruch 4 oder 5, **dadurch gekennzeichnet, dass** der Äquilibrierungspuffer ferner Trinatriumcitrat, Calciumchlorid, Glycin und Lysin mit einem pH-Wert zwischen 6,9 und 7,1 umfasst.

7. Verfahren nach Anspruch 6, **dadurch gekennzeichnet, dass** die Konzentrationen der Verbindungen des Äquilibrierungspuffers 0,01 M, 0,001 M, 0,12 M beziehungsweise 0,016 M betragen.

8. Verfahren nach einem der Ansprüche 1 bis 7, **dadurch gekennzeichnet, dass** der saure Waschpuffer aus einem Alkali- oder Erdalkalisalz von Essigsäure, Zitronensäure oder Phosphorsäure besteht in einer Konzentration zwischen 10 und 30 mM und einem pH-Wert zwischen 3,9 und 5,2.

9. Verfahren nach Anspruch 8, **dadurch gekennzeichnet, dass** der saure Waschpuffer einen 20 mM Natriumacetatpuffer pH 4,35 darstellt.

10. Verfahren nach einem der Ansprüche 1 bis 9, **dadurch gekennzeichnet, dass** der Durchfluss des Waschschritts b) einem Wert entspricht, der einen Faktor von ungefähr 1,5 bis 2 größer ist, als jener des Äquilibrierungsschritts a).

11. Verfahren nach einem der Ansprüche 1 bis 10, **dadurch gekennzeichnet, dass** in Schritt d) die Ionenstärke des Puffers aus Schritt c) durch Zugabe von Natriumchlorid erhöht wird, dessen Endkonzentration auf 0,15-0,17 M gebracht wird.

12. Verfahren nach einem der Ansprüche 1 bis 11, **dadurch gekennzeichnet, dass** es einen Schritt der viralen Inaktivierung der biologischen Fraktion durch ein Lösemittel-Detergens umfasst.

13. Verfahren nach einem der Ansprüche 1 bis 12, **dadurch gekennzeichnet, dass** es nach dem Eluierungsschritt d) einen oder mehrere der folgenden zusätzlichen Schritte umfasst:

- Sterilfiltration,
- Filtration zur Virusbeseitigung,
- Diafiltration,
- Aufkonzentrierung durch Ultrafiltration,
- Zugabe eines pharmazeutisch verträglichen Stabilisators,
- Lyophilisation,
- Trockenerhitzung zur Virusinaktivierung sowie
- Wiederherstellung des erhitzten Lyophilisats in einem wässrigen Medium, das für eine klinische Verwendung verträglich ist.

## Claims

1. Process for the preparation of a very high purity von Willebrand factor concentrate from a biological fraction containing von Willebrand factor, **characterized in that** it includes a separation by anion exchange chromatography using a vinyl polymer support of weak base type, the said separation comprising the steps of :

a) loading the chromatographic support with the fraction containing von Willebrand factor, previously equilibrated with a suitable buffer, with a predetermined flowrate, that allows the retention of the von Willebrand factor;
b) washing of the support with an acidic buffer with a flowrate higher than that of the step a) until the proteins and the not-retained contaminants are removed;
c) flushing and equilibrating of the chromatographic support with the buffer and using the flowrate of the step a); and
d) elution of the von Willebrand factor by increasing the ionic strength of the buffer of the buffer of step c).

2. Process according to claim 1, **characterized in that** the chromatographic separation is carried out on a support of vinyl polymer type grafted with anion exchange groups of DEAE.

3. Process according to claim 2, **characterized in that** the chromatographic support represents a copolymer of oligoethylene glycol, of glycidyl-methacrylate and of pentaerythritol methacrylate, on which are grafted anion exchange groups of weak base type, such as DEAE.

4. Process according to any one of claims 1 to 3, **characterized in that** the equilibration buffer of the step a) or c) is composed of sodium chloride.

5. Process according to claim 4, **characterized in that** the sodium chloride concentration is 0.11M.

6. Process according to claim 4 or 5, **characterized in that** the equilibration buffer further comprises trisodium citrate, calcium chloride, glycine and lysine, at a pH of 6.9-7.1.

7. Process according to claim 6, **characterized in that** the concentrations of the equilibration buffer components are of 0.01M, 0.001M, 0.12M and 0.016M respectively.

8. Process according to any one of claims 1 to 7, **characterized in that** the acidic washing buffer is composed of an alkaline or earth-alkaline salt of the acetic acid, citric acid or phosphoric acid, with a concentration in the range of between 10 and 30 mM and with a pH of 3.9-5.2.

9. Process according to claim 8, **characterized in that** the acidic washing buffer is a sodium acetate buffer 20 mM with a pH of 4.35.

10. Process according to any one of claims 1 to 9, **characterized in that** the flowrate of the washing step b) corresponds to a higher value by a factor of about 1.5 to 2 than that of the equilibration step a).

11. Process according to any one of claims 1 to 10, **characterized in that** in the step d), the ionic strength of the buffer of step c) is increased by adding sodium chloride final concentration of which is adjusted to 0.15-0.17M.

12. Process according to any one of claims 1 to 11, **characterized in that** it includes a step of virus inactivation treatment of the biological fraction by solvent-detergent.

13. Process according to any one of claims 1 to 12, **characterized in that** it includes, after the elution step d), one or more further steps consisting of

- sterile filtration,
- virus elimination filtration,
- diafiltration,
- concentrating ultrafiltration,
- addition of a pharmaceutically acceptable stabilizer,
- lyophilisation,
- virus inactivation by a dry heat treatment, and
- reconstitution of the heated lyophilisate with an aqueous medium suitable for clinical use.

EP 1 632 501 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0359593 A
- EP 0503991 A
- EP 0934748 A
- US 6579723 B
- EP 0383234 A
- US 6518482 B
- EP 359593 A
- FR 0308403

**Littérature non-brevet citée dans la description**

- **MAKRIS et al.** *Thromb.Haemost,* 2002, vol. 88, 377-378
- **MANUCCI P.M.** *Thromb.Haemost,* 2002, vol. 88, 378-379
- **COHN et al.** *J. Am. Chem. Soc.,* 1946, vol. 68, 459
- **KISTLER et al.** *Vox Sang.,* 1962, vol. 7, 414-424